# EUROPEAN PATENT APPLICATION

(11) **EP 4 129 287 A1**
(43) Date of publication of application: **08.02.2023**
(21) Application number: 21189699.8
(22) Date of filing: 04.08.2021
(51) Int. Cl.: A61K 31/4535, A61P 31/14

(54) **RALOXIFENE FOR USE IN THE TREATMENT OF SARS-COV-2 VARIANTS INFECTIONS**

(71) Applicant: Dompe' Farmaceutici S.P.A., 20122 Milan (IT); Istituto Nazionale per le Malattie Infettive "Lazzaro Spallanzani" I.R.C.C.S, 00149 Roma (IT)
(72) Inventor: BECCARI, Andrea Rosario, 80131 Napoli (IT); IACONIS, Daniela, 80131 Napoli (IT); TALARICO, Carmine, 80131 Napoli (IT); MANELFI, Candida, 80131 Napoli (IT); SCORZOLINI, Laura, 00149 Roma (IT); BORDI, Licia, 00149 Roma (IT); MATUSALI, Giulia, 00149 Roma (IT); NICASTRI, Emanuele, 00149 Roma (IT)
(74) Representative: Mauri, Elisabetta Maria Ester

(57) **Abstract**

The present invention relates to Raloxifene or a pharmaceutically acceptable salt thereof for use in the treatment of an infection caused by one or more variants of SARS-CoV-2 in a subject.

## Description

### FIELD OF THE INVENTION

The present invention relates to Raloxifene or a pharmaceutically acceptable salt thereof for use in the treatment of an infection caused by one or more variants of COVID-19 virus, SARS-CoV-2, in a subject.

### STATE OF THE ART

Coronaviruses (CoVs) are a large family of viruses belonging to the family *Coronaviridae.* The limited number of coronaviruses known to be circulating in humans were considered to cause mild infections and they were regarded in the past as relatively harmless respiratory human pathogens. However, in the last years the emergence of the severe acute respiratory syndrome coronavirus (SARS-CoV) and the Middle East Respiratory Syndrome (MERS) virus revealed that some coronaviruses can cause severe and sometimes fatal respiratory tract infections in humans (Pereira, H et al., 1989 Coronaviridae. Andrewes 'Viruses of Vertebrates, 5th ed.pp. 42-57; Holmes K.V., Lai M.M.C. Coronaviridae and their replication. In: Fields B.N., Knipe D.M., Howley P.M., editors. Fields Virology. 3rd edn. Lippincott-Raven; Philadelphia: 1996. p. 1075). The first known case of SARS-CoV occurred in Foshan, China in November 2002 and new cases emerged in mainland China in February 2003. The first emergence of MERS-CoV occurred in June 2012 in Saudi Arabia. These events demonstrated that the threats of CoVs should not be underestimated and that it is of paramount importance to advance the knowledge on the replication of these viruses and their interactions with the hosts to develop treatments and vaccines.

In December 2019, atypical pneumonia cases emerged in China and the cause was identified as being a novel coronavirus named SARS-CoV-2 by WHO.

Investigations of the epidemiological and clinical characteristics, and outcome of patients infected by SARS-CoV-2 showed that the infection causes clusters of severe respiratory illness similar to the known SARS-CoV. Furthermore, it was demonstrated that the SARS-CoV-2 can cause severe illness in some patients, even though initially it did not transmit readily between people. However, more recent epidemiological data suggest the new virus has undergone human host adaptation/evolution becoming more efficient in human-to-human transmission. Phylogenetic analysis of CoVs of different species indicated that SARS-CoV-2 could have originated from Chinese horseshoe bats, but the intermediate transmission vehicle has not yet been identified (Latinne A, et al. Origin and cross-species transmission of bat coronaviruses in China. Nat Commun. 2020 Aug 25;11(1):4235; Friend T et al., Future Virol. What is the intermediate host species of SARS-CoV-2? 2021 Mar: 10.2217/fvl-2020-0390. doi: 10.2217/fvl-2020-0390). According to this study, SARS-CoV-2 belongs to a novel type of bat coronavirus owing to a high degree of variation from the human SARS virus. SARS-CoV-2 is the seventh member of the family of CoVs that infects humans.

As for July 6^{th} 2021, SARS-CoV-2 infection led to more then 3.9 million deaths worldwide (https://covid19.who.int/). To date, with the advent of vaccine programs and constant social distancing interventions, it is believed that the virus is likely or very likely to become endemic. Nevertheless, the emerging variants of SARS-CoV-2 raise great concern for vaccine efficacy, events of reinfection and increased transmissibility and disease severity. As the virus started to spread around the world, a mutated spike SARS-CoV-2 variant (D614G), associated with increased transmissibility and infectivity, emerged and became predominant in Europe and worldwide although not accompanied by an increase in disease severity (Isabel S et al., Evolutionary and structural analyses of SARS-CoV-2 D614G spike protein mutation now documented worldwide Sci Rep 2020 Aug 20;10(1):14031; Koyama T et al., Variant analysis of SARS-CoV-2 genomes Bull World Health Organ. 2020 Jul 1; 98(7): 495-504; Korber B et al., Tracking Changes in SARS-CoV-2 Spike: Evidence that D614G Increases Infectivity of the COVID-19 Virus Cell 2020 Aug 20;182(4):812-827.e19; Mascola JR et al., SARS-CoV-2 Viral Variants-Tackling a Moving Target JAMA 2021 Apr 6;325(13):1261-1262). Until now other variants, which were defined as "variants of concern" (VOC), have emerged. The most relevant are: UK (B.1.1.7), South African (B.1.351), Brazilian P1 (B.1.1.28), Californian (B.1.427 and B.1.429, also named CAL.20C), that are all characterized by increased transmissibility, immune evasion and virulence (Funk T, et al. Characteristics of SARS-CoV-2 variants of concern B.1.1.7, B.1.351 or P.1: data from seven EU/EEA countries, weeks 38/2020 to 10/2021. Euro Surveill. 2021 Apr;26(16):2100348.; Davies NG, et al. Estimated transmissibility and impact of SARS-CoV-2 lineage B.1.1.7 in England. Science. 2021 Apr9;372(6538):eabg3055.; Moyo-Gwete T, et al. SARS-CoV-2 501 Y.V2 (B.1.351) elicits cross-reactive neutralizing antibodies. bioRxiv [Preprint]. 2021 Mar 6:2021.03.06.434193.; Tegally H, et al. Detection of a SARS-CoV-2 variant of concern in South Africa. Nature. 2021 Apr;592(7854):438-443.; Faria NR, et al. Genomics and epidemiology of the P.1 SARS-CoV-2 lineage in Manaus, Brazil. Science. 2021 May 21;372(6544):815-821; Sabino EC, et al. Resurgence of COVID-19 in Manaus, Brazil, despite high seroprevalence. Lancet. 2021 Feb 6;397(10273):452-455; Santos de Oliveira MH, et al. Sudden rise in COVID-19 case fatality among young and middle-aged adults in the south of Brazil after identification of the novel B.1.1.28.1 (P.1) SARS-CoV-2 strain: analysis of data from the state of Parana medRxiv [Preprint] Mer 2021; Deng X, et al. Transmission, infectivity, and antibody neutralization of an emerging SARS-CoV-2 variant in California carrying a L452R spike protein mutation. medRxiv [Preprint]. 2021 Mar 9:2021.03.07.21252647).

As of May 11, 2021, the Indian variant (Delta B.1.617.2) was added to the WHO list of VOC, and is estimated to be able to escape adaptive immunity induced by prior wildtype infection roughly half of the time and to be more infectious (around 60%) than the wildtype SARS-CoV-2 (Yang, W. et al. COVID-19 pandemic dynamics in India and impact of the SARS-CoV-2 Delta (B.1.617.2) variant. medRxiv 2021.06.21.21259268).

Therefore, there is currently an urgent need to identify new treatments able to exert antiviral activity against these new and clinically relevant SARS-CoV-2 variants.

The identification and development of new drugs is a lengthy process not adequate to face the emergency of the immediate global challenge of COVID-19 outbreak. Repurposing of drugs already tested as safe in man or approved for different therapeutic indications is a rapid response solution, since pharmacokinetic, toxicological, and manufacturing data for the drugs are already available, thus allowing immediate application in the new clinical setting (Jang WD, et al. Drugs repurposed for COVID-19 by virtual screening of 6,218 drugs and cell-based assay. Proc Natl Acad Sci USA. 2021 Jul 27;118(30):e2024302118.)

### SUMMARY OF THE INVENTION

The present inventors have found that Raloxifene exerts antiviral activity against the following SARS-CoV-2 variants: Wuhan, D614G, GV, VOC B.1.1.7, VOC P.1, VOC B.1.351 and VOC B.1.617.2.

Raloxifene is the generic name of 1-[6-hydroxy-2-(4-hydroxyphenyl)benzo[b]thien-3-yl]-1-[4-[2-(1-piperidinyl)ethoxy]phenyl]methanone, having the following general formula (I):

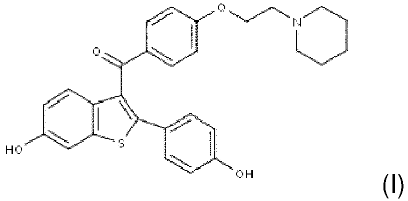

This compound is a selective benzothiophene estrogen receptor modulator (SERM) with lipid lowering effects and activity against osteoporosis and is approved for the treatment and prevention of osteoporosis in postmenopausal women, and in the USA also for the reduction of the risk of invasive breast cancer in postmenopausal women.

Raloxifene is therefore useful for the treatment of a subject having an infection caused by one or more SARS-CoV-2 variants selected from Wuhan, D614G, GV, VOC B.1.1.7, VOC P.1, VOC B.1.351 and VOC B.1.617.2.

Accordingly, a first object of the invention is Raloxifene or a pharmaceutically acceptable salt thereof for use in the treatment of an infection caused by at least one variant of SARS-CoV-2 in a subject, wherein said at least one variant is selected from Wuhan, D614G, GV, VOC B.1.1.7, VOC P.1, VOC B.1.351 and VOC B.1.617.2. Preferably, said subject has COVID-19.

A second object of the invention is a pharmaceutical composition comprising i) Raloxifene or a pharmaceutically acceptable salt thereof and ii) at least one inert pharmaceutically acceptable excipient, for use in the treatment of an infection caused by at least one variant of SARS-CoV-2 in a subject, wherein said at least one variant is selected from Wuhan, D614G, GV, VOC B.1.1.7, VOC P.1, VOC B.1.351 and VOC B.1.617.2. Preferably, said subject has COVID-19.

A third object of the invention is a method of treatment of an infection caused by at least one variant of SARS-CoV-2 in a subject, wherein said at least one variant is selected from Wuhan, D614G, GV, VOC B.1.1.7, VOC P.1, VOC B.1.351 and VOC B.1.617.2, the method comprising administering Raloxifene or a pharmaceutically acceptable salt thereof to the subject. Preferably, said subject has COVID-19.

### BRIEF DESCRIPTION OF THE FIGURES

Figure 1 shows the cytotoxity of Raloxifene at different concentrations (0.23 µM, 0.46 µM, 0.94 µM, 1.87 µM, 3.75 µM, 7.5 µM and 15 µM) on Vero E6 cells cultured in the presence of Raloxifene for 48 hours.
Figure 2 shows the cytotoxity of Raloxifene at different concentrations (0.23 µM, 0.46 µM, 0.94 µM, 1.87 µM, 3.75 µM, 7.5 µM and 15 µM) on Vero E6 cells cultured in the presence of Raloxifene for 72 hours.
Figure 3 shows the inhibition of cytopathic effect (CPE) induced by SARS-CoV-2 variant Wuhan in Vero E6 cells at different concentrations of Raloxifene. Percentage of viable cells calculated on not-infected-not-treated = 100%; infected-not-treated = 0%. Bars indicate SD.
Figure 4 shows the inhibition of cytopathic effect (CPE) induced by SARS-CoV-2 variant D614G in Vero E6 cells at different concentrations of Raloxifene. Percentage of viable cells calculated on not-infected-not-treated = 100%; infected-not-treated = 0%. Bars indicate SD.
Figure 5 shows the inhibition of cytopathic effect (CPE) induced by SARS-CoV-2 variant GV in Vero E6 cells at different concentrations of Raloxifene. Percentage of viable cells calculated on not-infected-not-treated = 100%; infected-not-treated = 0%. Bars indicate SD.
Figure 6 shows the inhibition of cytopathic effect (CPE) induced by SARS-CoV-2 variant VOC B.1.1.7 in Vero E6 cells at different concentrations of Raloxifene. Percentage of viable cells calculated on not-infected-not-treated = 100%; infected-not-treated = 0%. Bars indicate SD.
Figure 7 shows the inhibition of cytopathic effect (CPE) induced by SARS-CoV-2 variant VOC P.1 in Vero E6 cells at different concentrations of Raloxifene. Percentage of viable cells calculated on not-infected-not-treated = 100%; infected-not-treated = 0%. Bars indicate SD.
Figure 8 shows the inhibition of cytopathic effect (CPE) induced by SARS-CoV-2 variant VOC B.1.351 in Vero E6 cells at different concentrations of Raloxifene. Percentage of viable cells calculated on not-infected-not-treated = 100%; infected-not-treated = 0%. Bars indicate SD.
Figure 9 shows the inhibition of cytopathic effect (CPE) induced by SARS-CoV-2 variant VOC B.1.617.2 in Vero E6 cells at different concentrations of Raloxifene. Percentage of viable cells calculated on not-infected-not-treated = 100%; infected-not-treated = 0%. Bars indicate SD.

### DETAILED DESCRIPTION OF THE INVENTION

A first object of the present invention is Raloxifene or a pharmaceutically acceptable salt thereof for use in the treatment of an infection caused by at least one variant of SARS-CoV-2 in a subject, wherein said at least one variant is selected from Wuhan, D614G, GV, VOC B.1.1.7, VOC P.1, VOC B.1.351 and VOC B.1.617.2.

According to the present invention, the term "subject" refers to a human or animal being. Preferably, said subject is a human being.

According to the present invention, the subject having the infection to be treated may be symptomatic, paucisymptomatic or asymptomatic.

In a preferred embodiment, said subject has COVID-19. Thus, according to a preferred embodiment, Raloxifene or a pharmaceutically acceptable salt thereof for use according to the first object of the invention is for use in the treatment of COVID-19 in a subject. COVID-19 is the acronyme for "Coronavirus disease 2019", a contagious disease caused by SARS-CoV-2 and variants thereof. The disease is usually transmitted via the respiratory route when people inhale droplets and particles released by infected people ("Coronavirus disease (COVID-19): How is it transmitted?", www.who.int).

Symptoms of COVID-19 may include one or more of the following: fever, cough, headache, fatigue, breathing difficulties, and loss of smell and taste.

In an embodiment, the infection to be treated in the subject is caused by one variant of SARS-CoV-2 selected from Wuhan, D614G, GV, VOC B.1.1.7, VOC P.1, VOC B.1.351 and VOC B.1.617.2. In an alternative embodiment, the infection to be treated in the subject is caused by two or more variants of SARS-CoV-2 selected from Wuhan, D614G, GV, VOC B.1.1.7, VOC P.1, VOC B.1.351 and VOC B.1.617.2.

The identification of an infection caused by one or more of the variants of SARS-CoV-2 listed above in a subject is usually based on the analysis of the presence of viral RNA in biological material from the subject, preferably specimens from upper and lower respiratory tract, such as nasopharyngeal/oropharyngeal swabs, nasal aspirate, nasal wash or saliva, sputum, tracheal aspirate or bronchoalveolar lavage.

As used herein the term "pharmaceutically acceptable salt" of Raloxifene refers to an addition salt of this compound with an acid or a base that forms non-toxic acid anions or cations.

Preferably, said pharmaceutically acceptable salt of Raloxifene is Raloxifene hydrochloride.

As will be described in detail in the experimental section, the present inventors have found that, in addition to its known activity, Raloxifene exerts antiviral activity against the following variants of SARS-CoV-2: Wuhan, D614G, GV, VOC B.1.1.7, VOC P.1, VOC B.1.351 and VOC B.1.617.2.

Being an already marketed drug, Raloxifene has the advantage that it has already been approved for clinical use and tested as safe in humans, and can therefore provide a rapid therapeutic approach to the treatment of a subject as above defined who has an infection caused by one or more variants of SARS-CoV-2 listed above.

According to the first object of the invention, Raloxifene or a pharmaceutically acceptable salt thereof is administered in form of a pharmaceutical composition where it is admixed with one or more pharmaceutically acceptable excipients.

Thus, a second object of the invention is a pharmaceutical composition comprising Raloxifene or a pharmaceutically acceptable salt thereof and at least one inert pharmaceutically acceptable excipient, for use in the treatment of an infection caused by at least one variant of SARS-CoV-2 in a subject as above defined, wherein said at least one variant is selected from Wuhan, D614G, GV, VOC B.1.1.7, VOC P.1, VOC B.1.351 and VOC B.1.617.2.

In a preferred embodiment, said subject has COVID-19. Thus, according to a preferred embodiment the pharmaceutical composition for use according to the second object of the invention is for use in the treatment of COVID-19 in a subject.

The route of administration of Raloxifene or of the pharmaceutically acceptable salt thereof for use according to the present invention is in accordance with known methods of administration of this compound, which is usually by systemic administration, preferably by oral, parenteral or inhalatory route. The term "parenteral" as used herein includes intravenous, intraperitoneal, intracerebral, intrathecal, intracranial, intramuscular, intraarticular, intrasynovial, intrasternal, intraocular, intraarterial, subcutaneous, intracutaneous injection or infusion techniques.

The pharmaceutical composition for use according to the present invention may be formulated into oral, inhalatory or injectable dosage forms such as tablets, capsules, powders, solutions, suspensions, and emulsions.

Preferably, the pharmaceutically acceptable excipient in the pharmaceutical composition includes any and all solvents, diluents, or other vehicles, dispersion or suspension aids, surface active agents, isotonic agents, thickening or emulsifying agents, preservatives, solid binders, lubricants and the like, as suited to the particular dosage form desired.

Some examples of materials which can serve as pharmaceutically acceptable excipient include, but are not limited to, sugars such as lactose, glucose and sucrose; starches such as corn starch and potato starch; cellulose and its derivatives such as sodium carboxymethyl cellulose, ethyl cellulose and cellulose acetate; powdered tragacanth; malt; gelatine; talc; cocoa butter and suppository waxes; oils such as peanut oil, cottonseed oil, safflower oil, sesame oil, olive oil, corn oil and soybean oil; glycols, such a propylene glycol; esters such as ethyl oleate and ethyl laurate; agar; buffering agents; alginic acid; pyrogen-free water; salts for regulating osmotic pressure; sterilized water; ethyl alcohol; preservatives, stabilizers, surfactants, emulsifiers, sweeteners, colorants, flavourings and the like.

The pharmaceutical composition of the present invention may be suitably formulated using appropriate methods known in the art or by the method disclosed in Remington's Pharmaceutical Science (recent edition), Mack Publishing Company, Easton Pa.

In a preferred embodiment, the invention provides for oral administration of the compound or of the pharmaceutical composition for use according to the invention.

In such embodiment, the pharmaceutical composition is preferably in form of a tablet or a capsule.

According to an alternative embodiment, the compound or pharmaceutical composition for use according the invention is administered parenterally, preferably by intravenous administration or continuous infusion. This route of administration is particularly suitable for intubated or critically ill COVID-19 patients.

In yet an alternative embodiment, the invention provides for direct administration of the compound or pharmaceutical composition for use according to the present invention to the respiratory tract. The direct administration to the respiratory tract may be the sole route of administration of the compound or composition or may be combined with administration of the compound or composition via other systemic routes.

For direct administration of the compound or composition for use according to the invention, a wide variety of devices that are designed for the direct delivery of pharmaceutical compositions and therapeutic formulations to the respiratory tract may be used. In one aspect of the present invention, the compound or pharmaceutical composition of the present invention is administered in aerosolized or inhaled form. The pharmaceutical composition for use according to the present invention for direct administration to the respiratory tract as described above, can be in form of an aerosol formulation, as a dry powder or as a solution or suspension in a suitable diluent.

The dosage regimen of Raloxifene or a pharmaceutically acceptable salt thereof for use according to the present invention is that usually employed for other indications of Raloxifene.

The dose and frequency of administration of Raloxifene or a pharmaceutically acceptable salt thereof for use according to the present invention will vary with the pharmaceutically-acceptable excipient(s)/carrier(s) utilized, the severity of the condition being treated, the patient's age, body weight, general health status and sex, the chosen route of administration and dosage form, the number of administrations per day, the duration of the treatment, the nature of possible concurrent therapies and other factors within the knowledge and expertise of the attending physician. A person skilled in the art can determine the optimum posology on the base of the known pharmacokinetic properties and dosage regime of the compound.

In a preferred embodiment, Raloxifene or a pharmaceutically acceptable salt thereof, preferably Raloxifene hydrochloride, is administered orally to the subject to be treated at a dosage comprised between 50 and 150 mg per day. More preferably, the dosage of Raloxifene or of the pharmaceutically acceptable salt thereof is selected from 60 mg per day and 120 mg per day.

When Raloxifene or a pharmaceutically acceptable salt thereof is administered at a dosage of 120 mg per day, it can be administered in one or two administrations. The total daily dosage may therefore be administered in a single 120 mg dose or may be divided in two daily 60 mg doses.

As the skilled artisan will appreciate, lower or higher doses than those recited above may be required depending on specific istances.

A third object of the invention is a method of treating an infection caused by at least one variant of SARS-CoV-2 in a subject as above defined, wherein said at least one variant is selected from Wuhan, D614G, GV, VOC B.1.1.7, VOC P.1, VOC B.1.351 and VOC B.1.617.2, the method comprising administering Raloxifene or a pharmaceutically acceptable salt thereof, preferably the hydrochloride salt, to said subject.

In an embodiment, said method comprises the steps of:
a) identifying a subject having an infection caused by one or more variants of SARS-CoV-2 selected from Wuhan, D614G, GV, VOC B.1.1.7, VOC P.1, VOC B.1.351 and VOC B.1.617.2; and
b) administering to the subject identified in step a) Raloxifene or a pharmaceutically acceptable salt thereof.

According to a particularly preferred embodiment of the method of treating an infection caused by at least one variant of SARS-CoV-2 as above described, step a) is carried out basing on an analysis of the presence of viral RNA in biological material obtained from the subject. Preferably, said biological material is selected from one or more of: specimens from upper and lower respiratory tract, such as nasopharyngeal/oropharyngeal swabs, nasal aspirate, nasal wash or saliva, sputum, tracheal aspirate or bronchoalveolar lavage.

In a preferred embodiment, said subject has COVID-19. Thus, according to a preferred embodiment, the method according to the third object of the invention is a method of treating COVID-19 in a subject.

The invention will be further described in the following examples, which do not limit the scope of the invention described in the claims.

### EXPERIMENTAL PART

### EXAMPLE 1

### Evaluation of antiviral activity of Raloxifene on SARS-CoV-2 variants

The evaluation of the antiviral activity of Raloxifene against various SARS-CoV-2 variants was carried out on African green monkey kidney Vero E6 cell line. This cell line was obtained from American Type Culture Collection (ATCC, Manassas, VA, USA) and maintained in Dulbecco's Modified Eagle Medium (DMEM; Gibco, Thermo-Fisher, Waltham, MA, USA) supplemented with 10% fetal bovine serum (FBS; Gibco, Thermo-Fisher) at 37°C in a humidified atmosphere of 5% CO2.

The following SARS-CoV-2 variants were used to infect the cells:
- Human 2019-nCoV variant 2019-nCoV/Italy-INMI1, clade V (Ref-SKU: 008V-03893, EVAg portal), named Wuhan, isolated in January 2020 from a Chinese patient (control infection). The sequence is available on GISAID: EPI_ISL_410545;
- SARS-CoV-2 isolate SARS-CoV-2/Human/ITA/PAVIA10734/2020, clade G, D614G (S) (Ref-SKU: 008V-04005, EVAg portal), named D614G, isolated in Lombardy in February 2020. The sequence is available on GISAID: EPI_ISL_568579;
- SARS-CoV-2 isolate hCoV-19/Italy/LAZ-INMI-82isl/2020, clade GV, A222V, D614G (S) (Ref-SKU: 008V-04048, EVAg portal), named GV and representing the dominant variant circulating in Europe from April to December 2020. The sequence is available on GISAID: EPI_ISL_824408;
- SARS-CoV-2 variant VOC 202012/01, isolate hCoV-19/Italy/CAM-INMI-118isl/2020, clade GR, Δ69-70, Δ144, N501Y, A570D, D614G, P681H, T716I (S) (Ref-SKU: 008V-04050, EVAg portal), named VOC B.1.1.7 and representing the variant of major concern from UK. The sequence is available on GISAID: EPI_ISL_913449;
- SARS-CoV-2 variant GR/501Y.V3, isolate hCoV-19/Italy/LAZ-INMI-216isl/2021, clade GR, PANGO lineage P.1, K417T, E484K, N501Y (S) (Ref-SKU: 008V-04101, EVAg portal), named VOC P.1 and representing the variant of major concern from Brazil. The strain was isolated from the biological sample that contains SARS-CoV-2 variant GR/501Y.V3, hCoV-19/Italy/LAZ-INMI-216/2021 strain. The sequence is available on GISAID: EPI_ISL_1023524;
- SARS-CoV-2 variant VOC SA/B.1.351, clinical isolate obtained by GHSAG (Public Health England), named VOC B.1.351 and representing the variant of major concern from South Africa;
- SARS-CoV-2 variant VOC G Delta /B1.617.2 isolate hCoV-19/Italy/LAZ-INMI-648/2021, named VOC B.1.617.2, representing the variant of major concern from India. The sequence is available on GISAID: EPI_ISL_2000624.

First, the time-window in which cytopatic effect (CPE) appeared for each variant was determined. The CPE was evident at 48 h for all the tested variants but VOC B.1.1.7 and VOC P1 variants, for which evident CPE appeared later (56h and 72h, respectively).

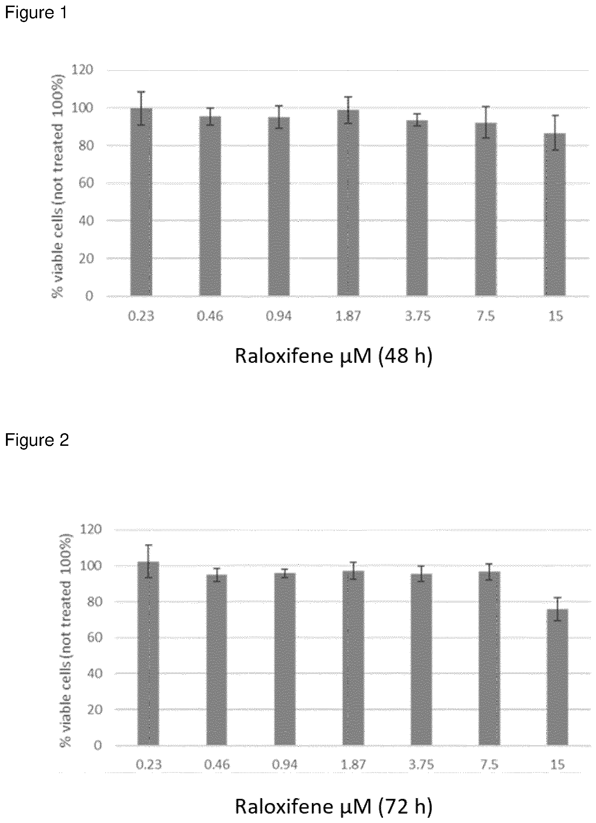

In parallel, not infected cells were cultured in the presence of different doses of Raloxifene to evaluate possible cytotoxicity due to the treatment. Cells were seeded into 24-well plates (2.5x104 cells/well) in DMEM supplemented with 10% FBS, and treated with different doses of Raloxifene (0.23 µM, 0.46 µM, 0.94 µM, 1.87 µM, 3.75 µM, 7.5 µM and 15 µM). Results are shown in Figure 1 and Table 1 below (evaluation at 48 h) and Figure 2 and Table 2 below (evaluation at 72 h).

**Table 1**

| **Raloxifene µM (48 h)** | **% viable cells** | **St dev** |
|---|---|---|
| 0.23 | 100 | 8.6 |
| 0.46 | 95 | 4.6 |
| 0.94 | 95 | 6.0 |
| 1.87 | 99 | 7.0 |
| 3.75 | 93 | 3.2 |
| 7.5 | 92 | 8.4 |
| 15 | 86 | 9.2 |

**Table 2**

| **Raloxifene µM (72 h)** | **% viable cells** | **St dev** |
|---|---|---|
| 0.23 | 102 | 9.0 |
| 0.46 | 95 | 3.8 |
| 0.94 | 96 | 2.5 |
| 1.87 | 97 | 4.8 |
| 3.75 | 96 | 4.4 |
| 7.5 | 97 | 4.6 |
| 15 | 76 | 6.6 |

In cells treated with Raloxifene 15 µM, a reduced percentage of viable cells was observed as revealed by crystal violet staining. No significant effect on cell viability was observed with lower dug concentrations (7.5 to 0.23 µM).

To determine the antiviral efficacy of the drug, Vero E6 cells were infected at 37°C for 1 h each with a single variant of the SARS-CoV-2 variants listed above at a MOI of 0.05 in 96 well plates. Infection was carried out in MEM (Sigma) without FBS (Gibco). Then, the virus was removed and cells washed with warm phosphate buffered saline (PBS) and cultured with medium containing 2% FBS in the presence or absence of Raloxifene at different doses (0.23, 0.47, 0.94, 1.88, 3.75, 7.5, 15 µM) at 37°C and 5% CO2 up to 72 h.

To determine the antiviral efficacy of Raloxifene, cell viability and viral induced cytopathic effect (CPE) were measured both in not infected and infected cells treated with serial dilution of the drug and staining the cells with a solution of Crystal Violet (Diapath) and 2% formaldehyde. After 30 min, the fixing solution was removed by washing with tap water, and cell viability was measured by a photometer at 595 nm (Synergy^{™} HTX MultiMode Microplate Reader, Biotek, Winooski, VT, USA).

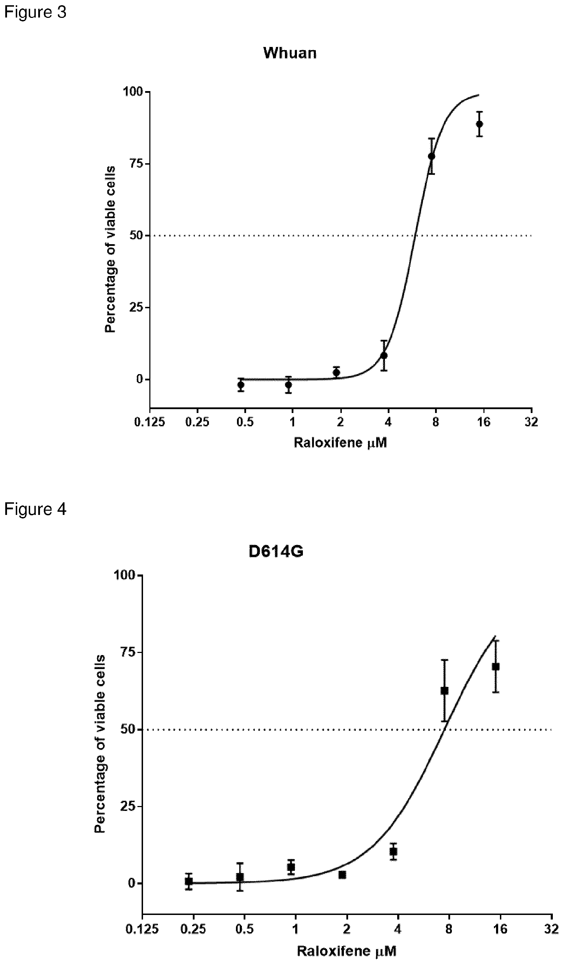

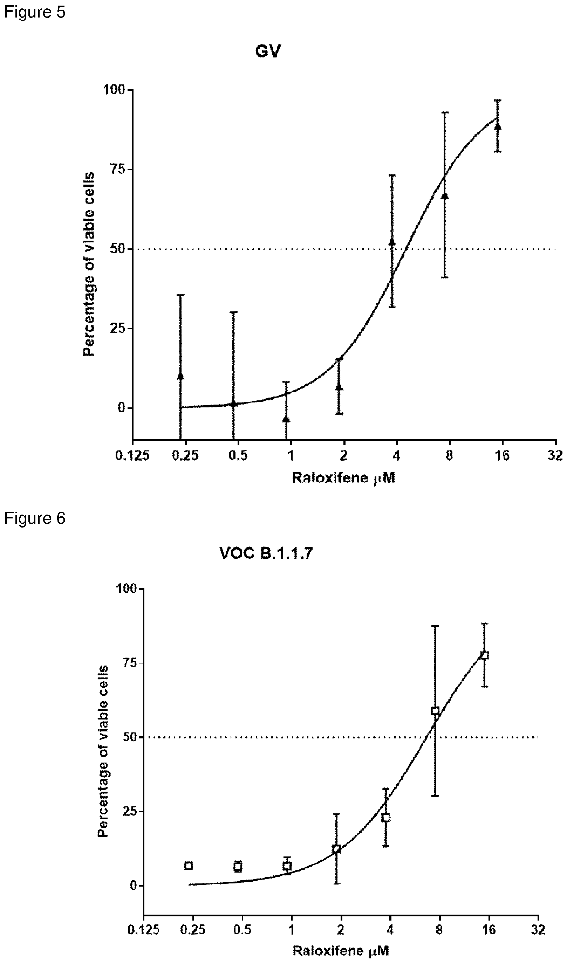

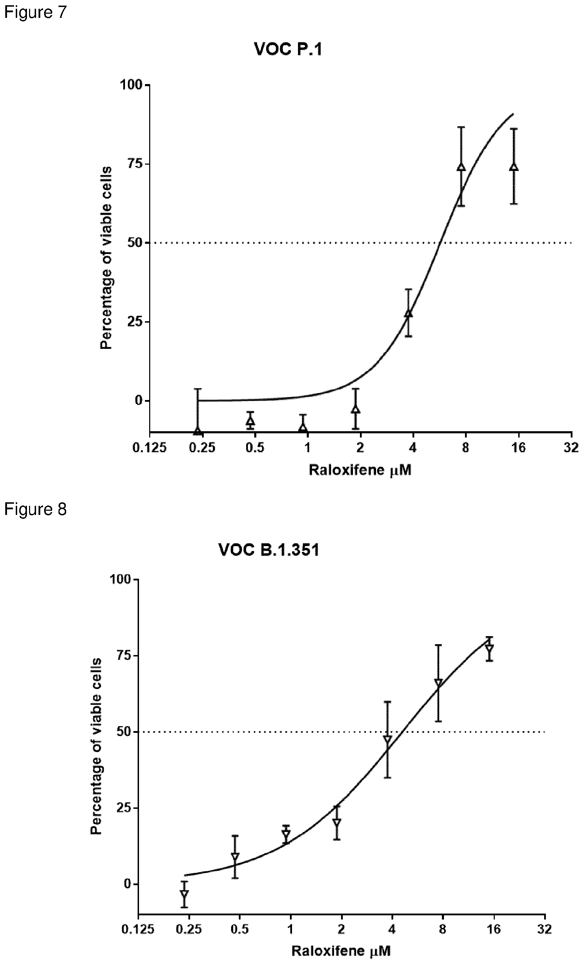

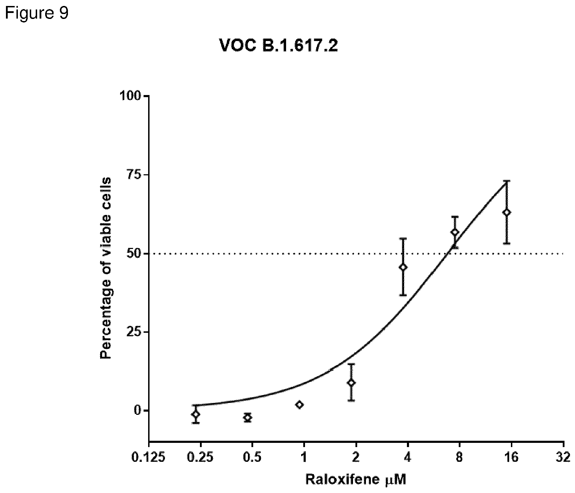

The percentage of viable cells for each condition was calculated compared to infected-not-treated (set as 0%) and not-infected-not-treated cells (set as 100%). The results are shown in Figures 3 to 9. The effect of Raloxifene on cell viability was determined with the same methods in each experiment performed for SARS-CoV-2 variants study.

As can be seen, Raloxifene was able to recover viability in Vero E6 cells infected with all the tested viral variants.

The half-maximal inhibitory concentration (IC50) for Raloxifene was calculated from concentration-effect-curves after non-linear regression analysis using GraphPad Prism8. The results are indicated in Table 3.

**Table 3**

| **Variant** | **IC50 (µM)** | **95% CI** |
|---|---|---|
| Wuhan | 5.92 | 5.29-6.54 |
| D614G | 7.48 | 6.38-8.90 |
| GV | 4.51 | 3.22-6.47 |
| VOC B.1.1.7 | 6.65 | 5.25-8.54 |
| VOC P.1 | 5.70 | 4.61-7.22 |
| VOC B.1.351 | 4.58 | 3.81-5.55 |
| VOC B.1.617.2 | 7.99 | 5.91-11.68 |

As can be seen, the IC50 calculated on recovering of cell viability varied from 4.50 to 7.99 µM depending on the variant tested, showing a strong antiviral activity of Raloxifene against all the variants under investigation.

## Claims

1. Raloxifene or a pharmaceutically acceptable salt thereof for use in the treatment of an infection caused by at least one variant of SARS-CoV-2 in a subject, wherein said at least one variant is selected from Wuhan, D614G, GV, VOC B.1.1.7, VOC P.1, VOC B.1.351 and VOC B.1.617.2.

2. Raloxifene or a pharmaceutically acceptable salt thereof for use according to claim 1, wherein said subject has COVID-19.

3. Raloxifene or a pharmaceutically acceptable salt thereof for use according to claim 2, wherein Raloxifene or said pharmaceutically acceptable salt thereof is for use in the treatment of COVID-19.

4. Raloxifene or a pharmaceutically acceptable salt thereof for use according to any one of claim 1 to 3, wherein said pharmaceutically acceptable salt is Raloxifene hydrochloride.

5. Raloxifene or a pharmaceutically acceptable salt thereof for use according to any one of claims 1 to 4, wherein Raloxifene or said pharmaceutically acceptable salt thereof is administered to said subject orally at a dosage comprised between 50 mg per day and 150 mg per day.

6. Raloxifene or a pharmaceutically acceptable salt thereof for use accoding to any one of claims 1 to 5, wherein Raloxifene or said pharmaceutically acceptable salt thereof is administered to said subject orally at a dosage selected from 60 mg per day and 120 mg per day.

7. A pharmaceutical composition comprising:
i) Raloxifene or a pharmaceutically acceptable salt thereof, and
ii) at least one inert pharmaceutically acceptable excipient,
for use in the treatment of an infection caused by at least one variant of SARS-CoV-2 in a subject, wherein said at least one variant is selected from Wuhan, D614G, GV, VOC B.1.1.7, VOC P.1, VOC B.1.351 and VOC B.1.617.2.

8. A pharmaceutical composition for use according to claim 7, wherein said pharmaceutical composition is administered orally.

9. Raloxifene or a pharmaceutically acceptable salt thereof for use according to claim 5 or claim 6 or a pharmaceutical composition for use according to claim 8, in form of a tablet or capsule.

10. Raloxifene or a pharmaceutically acceptable salt thereof for use according to any one of claims 1 to 4 or a pharmaceutical composition for use according to claim 7, wherein Raloxifene or said pharmaceutically acceptable salt thereof or said pharmaceutical composition is administered parenterally.

11. Raloxifene or a pharmaceutically acceptable salt thereof or a pharmaceutical composition for use as claimed in claim 10, wherein Raloxifene or said pharmaceutically acceptable salt thereof or said pharmaceutical composition is administered by intravenous administration or continuous infusion.

12. Raloxifene or a pharmaceutically acceptable salt thereof for use according to any one of claims 1 to 4 or a pharmaceutical composition for use according to claim 7, wherein Raloxifene or said pharmaceutically acceptable salt thereof or said pharmaceutical composition is administered by direct administration to the respiratory tract, optionally in combination with other systemic routes.
